# EUROPEAN PATENT APPLICATION

(11) **EP 2 796 541 A1**
(43) Date of publication of application: **29.10.2014**
(21) Application number: 12855702.2
(22) Date of filing: 07.12.2012
(51) Int. Cl.: C12M 3/00, C12N 5/00

(54) **IN-VITRO CELL COLONY CULTIVATION DEVICE AND USE THEREOF**

(30) Priority: 07.12.2011 CN 201110403987
(71) Applicant: Tsinghua University, Beijing 100084 (CN)
(72) Inventor: DU, Yanan, Beijing 100084 (CN); YAO, Rui, Beijing 100084 (CN); WANG, Jingyu, Beijing 100084 (CN)
(74) Representative: Schwarz & Partner
(86) International application number: PCT/CN2012/086149
(87) International publication number: WO 2013/083073

(57) **Abstract**

Provided are an in-vitro cell colony culture device and use thereof, the in-vitro cell colony culture device comprising: a micro-patterned template having a micro-patterned cavity, and adhesive hydrogel formed on the lower surface of the micro-patterned template, and the micro-patterned cavity defining the growth space of the cell colony. An upper template can be retained or removed as required to from a monolayer cell micro-pattern or a multiplayer cell micro-cluster.

## Description

### CROSS-REFERENCE TO RELATED APPLICATION

This application claims priority to and benefits of Chinese Patent Application Serial No. 201110403987.8, filed with the State Intellectual Property Office of P. R. China on July 12, 2011, the entire content of which is incorporated herein by reference.

### FIELD

The present disclosure relates to an in-vitro cell colony culture device and use thereof. Particularly, the present disclosure relates to an in-vitro cell colony culture device and use thereof in regulating the self-renewal and directed differentiation of stem cell, as well as a method for regulating the self-renewal of stem cell, a method for regulating the directed differentiation of stem cells, a method for forming a monolayer micro-pattern of cell, and a method for forming a multilayer micro-cluster of cell.

### BACKGROUND

Totipotent stem cell (TSC) refers to a stem cell having an infinite differentiation potential, which may be able to differentiate into all kinds of tissues and organs, and the TSC mainly comprises embryonic stem cell (ESC) and induced pluripotent stem cells (iPS). The embryonic stem cell is obtained by isolating and culturing from early embryo of mammals, having a characteristic of developmental totipotent, involving the whole development process of organism, and constituting various tissues and organs of human body. The embryonic stem cells are obtained from blastocyst at a gastrula stage, which may further differentiate into multipotent, such as hepatic progenitor cells, bone marrow hematopoietic stem cells and etc. From a technical angle, an embryonic stem cell being totipotent is the most optimal technique for therapeutic cloning. However, as referring to ethics and morality, study on human embryonic stem cell is prohibited by law in many countries, and relative research is in a dilemma. In 2007, it was announced respectively by Japanese and American scientists that a method of transferring an ordinary skin cell into a stem cell has been found out, and the stem cell obtained thereof possesses a similar function with the embryonic stem cell, which is known as induced pluripotent stem cells, *i.e.* iPS cell. This discovery has been regarded as the great progress of science by two authoritative science magazine, *Science* and *Nature,* in that year.

iPS is an ordinary somatic cell after "initialization", which has a similar function with embryonic stem cell and can differentiated into various tissue cells. More importantly, it has overcome a lot of obstacles such as ethics and laws faced by study of embryonic stem cell all the time, thus the application prospect in medical field thereof is very broad.

Many countries such as America, Japan and *et al* promote such emerging stem cell research even with more enthusiasm, by increasing investments or establishing encouragement policies. The fundamental research and applied research of stem cell in China start early, from 2001, there are no fewer than 20 major studies relative to stem cell. In addition, state has also given great support in research & development of network construction and talent construction, as well as in building of research platform, and *et al,* which has laid foundation in such research field for China with an ascendant position. Mouse have been cloned with iPS cell, respective by ZHOU Qi (Institution of Zoology, Chinese Academy of Sciences) and GAO Shaorong (National Institute of Biological Sciences, Bejing), accordingly the totipotency of iPS cell has been proved for the first time in the world, suggesting that cell having the same differentiative capacity with embryonic stem cell may be obtained thoroughly by means of in vitro manipulation. This achievement is one giant leap from stem cell study towards actual medical procedure, which means a great value for totipotent mechanism and clinical application study (such as organ transplantation, drug screening, gene therapy and *et al*) of stem cell.

Colony growth of totipotent stem cell plays a very important role in maintaining the totipotency of stem cell. Taken human embryonic stem cell (hESC) as an example, a classic cultural method is conducted on an inactivated feeder cell represented by mouse embryonic fibroblasts (MEF), meanwhile it has been proven by some studies that, under an effect of suitable chemical factors, an extracellular matrix may also act as a culture substrate to maintain self-renewal of hESC, such as one of matrigel, collagen, laminin and fibronectin or a mixture thereof. In a condition of single cell, the survival rate of hESC is relative low, in consequence a classic method of passaging cells is that the colony is digested or cutting into clusters having about 100 cells in each cluster. Regardless of any methods for passaging cell, the key operation is to maintain hESC in the state of cell clusters. However, such method depends on experiences and skills of operating personnel to a great extent, which results in finally forming a heterogeneous hESC colony with random shape and size, as well as random density, while cell cluster oversized or undersized has a more obvious trend of differentiation. For the special requirement for self-renewal of totipotent stem cell, it is difficult for current method of culturing and passaging cells to obtain a colony of homogeneous totipotent stem cell.

At present, it has been proven by more and more studies that the homogeneity of totipotent stem cell colony plays a fatal role in self-renewal and subsequent differentiation. Taken hESC as an example, the size of colony and the composition of cells have a regulating effect for inducing factor of differentiation and maintaining factor of totipotency, particularly, Smad1 signal directly correlated to colony size is activated by antagonisms of embryonic stem cell and embryonic endodermal cell derived thereof, which mediating interaction between bone morphogenetic protein -2 (BMP2) secreted by the embryonic endodermal cell, and growth differentiation factor -3 (GDF3) secreted by the embryonic stem cell. The auto-differentiation of embryonic stem cell may be rescued by regulation of Smad 1 activation alone, indicating that the hESC colony size may independently regulate differentiation and self-renewal of the stem cell. While the colony size cannot be adjusted under a classic culturing condition, there must exists an uncontrollable auto-differentiation under coaction of random colony size, random cell density and molecule microenvironment, which may affect proliferation and self-renewal of stem cell.

The heterogeneity and size difference of the totipotent stem cell colony also play crucial roles in differentiation fate of stem cell. Uncontrollable colony of initial stem cell usually leads to inconsistent or unstable result and efficiency of differentiation, which is the general problem faced by differentiation study of totipotent stem cell. It has been found out by micropatterning technique that, a colony with a relative smaller size has a more obvious trend of differentiating towards cardiomyocyte, while a colony with a relative larger size has a more obvious trend of differentiating towards neurocyte. Mechanical property of culture substrate of totipotent stem cell is also important for self-renewal and differentiation fate of the stem cell. It has been proven by study that the culture substrate, with an elasticity modulus of 0.6 kPa, has an excellent maintaining effect on self-renewal of mouse embryonic stem cell. The mentioned mechanical property is similar with in vivo tissue; similarly, under the condition without adding chemical signal stimulation, a culture substrate with a relative higher mechanical strength has an effect on inducing mesenchymal stem cells to differentiate towards osteoblast, while a culture substrate with a relative lower mechanical strength may induce stem cell to differentiate towards adipocyte. It has been proven by much deeper study that suitable mechanical stress may suppress auto-differentiation of hESC, and promote self-renewal of hESC, while the synergy of bi-cyclic stress and chemical signal may regulate the auto-differentiation and the self-renewal of hESC, which is of significant for research on fields such as stem cell differentiation and cell therapy.

After 2011, it has been proven by research achievement published in Journals such as *Nature* and *Cell Stem Cell* by Yoshiki Sasain and *et al.* that, totipotent stem cell, such as hESC and mouse embryonic stem cell, is subjected to directed differentiation under a condition of three-dimensional cell cluster culture, which may obtain similar to retinal tissue having natural multi-layered structure in vitro, whose development process has a multifaceted similarity comparing with natural tissue in vivo. Such method and achievement of research has great advantages than the traditional two-dimensional study, manifesting a huge potential in directed inducing to differentiate into totipotent stem cell under the condition of three-dimensional cell-cluster culture.

Thus, the most optimal platform for culturing totipotent stem cell shall comprise following factors of four aspects under a premise of maintaining normal phenotype and karyotype of stem cell: controllable shape and size of colony, culture substrate with adjustable physical and chemical properties, various culturing patterns such as achievable monolayer cell colony and multilayer cell cluster, suitable passaging method, so as to realize an adjustable and stable cell seeding density.

### SUMMARY

The present disclosure directs to solve at least one of the problems existing in the prior art to at least some extent. Thus, one purpose of the present disclosure is to provide an apparatus for cell colony culture *in vitro* with controllable shape and size of colony, culture substrate with adjustable physical and chemical properties, various culturing patterns such as achievable monolayer cell colony and multilayer cell cluster, suitable passaging method.

According to a first aspect of the present disclosure, there is provided an apparatus for cell colony culture *in vitro.* According to embodiments of the present disclosure, the apparatus for cell colony culture *in vitro* may comprise: a micro-patterned plate having a micro-patterned cavity; an adhesive hydrogel formed on a lower surface of the micro-patterned plate, in which the micro-patterned cavity defines a growth space for cell colony. The inventors surprisingly find out that using the apparatus for cell colony culture *in vitro* of the present disclosure may achieve a homogeneous growth of cell colony. In particular, according to embodiments of the present disclosure, the apparatus for cell colony culture *in vitro* of the present disclosure, integrally using the micro-patterned plate and the adhesive hydrogel, may achieve regulations on various factors, such as shape and size of cell colony, cell growth manner in monolayer or multilayer, mechanical strength and chemical component of adhesive substrate, growth factor and chemical signal, co-cultured cell; while the regulations to various factors are independent without mutual interference; may achieve proliferation and induced differentiation of cell *in situ,* which greatly simplifies the operations, and can reduce treatment and damage to cell; may keep an excellent sterile environment without changing original operating steps and details of cell culture by together using a commercial culture dish, which makes the preparation thereof simple, convenient and easy; may conveniently construct various cell co-culturing environment at the same time by taking full advantage of micro-patterned technique, which provides convenient and practical method for researches of model construction and screening with high-throughput and high-content.

According to embodiments of the present disclosure, the apparatus for cell colony culture *in vitro* may also have following additional technical features:

According to an embodiment of the present disclosure, the apparatus for cell colony culture *in vitro* may further comprise a hard substrate, located on a lower surface of the adhesive hydrogel.

According to an embodiment of the present disclosure, the apparatus for cell colony culture *in vitro* may further comprise a multi-well culture plate, located under the adhesive hydrogel and embedded in the micro-patterned plate.

According to an embodiment of the present disclosure, the adhesive hydrogel is formed by cross-linking at least one selected from a group consisting of a natural biomaterial and a synthetic biomaterial.

According to an embodiment of the present disclosure, the natural biomaterial is at least one selected from a group consisting of gelatin, gelatin derivative, alginate, alginate derivative, agar, matrigel, collagen, proteoglycan, glycoprotein, hyaluronic acid, laminin and fibronectin.

According to an embodiment of the present disclosure, the synthetic biomaterial is at least one selected from a group consisting of polypropylene, polystyrene, polyacrylamide, polylactic, poly-hydroxyl acid, polylactic-alkyd copolymer, poly-dimethylsiloxane, polyanhydride poly-acid-ester, polyamide, polyamino acid, polyacetal, polycyanoacrylate, polyurethane, polypyrrole, polyester, polymethacrylate, polyethylene, polycarbonate and polyepoxyethane.

According to an embodiment of the present disclosure, the micro-patterned plate is formed by poly(methyl methacrylate).

According to an embodiment of the present disclosure, the adhesive hydrogel is formed from gelatin-methyl methacrylate and a photoinitiator, and the photoinitiator is 2-hydroxy-4-(2-hydroxyethoxy)-2-methyl propiophenon.

According to an embodiment of the present disclosure, the gelatin-methyl methacrylate is a polymer of gelatin and methyl methacrylate at a ratio of 1 g of gelatin against 1 mL of methyl methacrylate.

According to an embodiment of the present disclosure, the adhesive hydrogel is prepared by the following steps: dissolving gelatin-methyl methacrylate in DMEM medium to obtain a gelatin-methyl methacrylate solution, in which a content of gelatin-methyl methacrylate in the gelatin-methyl methacrylate solution is 5 g of gelatin- methyl methacrylate per 100 mL of the gelatin-methyl methacrylate solution; dissolving the photoinitiator in absolute ethanol to obtain a photoinitiator solution, in which a content of the photoinitiator in the photoinitiator solution is 10 g of the photoinitiator per 100 mL of the photoinitiator solution; mixing the photoinitiator solution and the gelatin-methyl methacrylate solution at a volume ratio of 1:20; and cross-linking gelatin-methyl methacrylate using ultraviolet ray to obtain the adhesive hydrogel.

According to an embodiment of the present disclosure, the adhesive hydrogel comprises at least one selected from a group consisting of extracellular matrix component, growth factor and chemical signaling molecule.

According to an embodiment of the present disclosure, the extracellular matrix is coated on an upper surface of the adhesive hydrogel.

According to an embodiment of the present disclosure, the extracellular matrix component is at least one selected from a group consisting of collagen, gelatin, matrigel, proteoglycan, glycoprotein, hyaluronic acid, laminin and fibronectin.

According to an embodiment of the present disclosure, the adhesive hydrogel is fitted with the growth factor and the chemical signal molecule.

According to an embodiment of the present disclosure, the adhesive hydrogel has a thickness of 1-100 µm.

According to another aspect of the present disclosure, there is provided a usage of the above-mentioned apparatus for cell colony culture *in vitro* in regulating the self-renewal of stem cell.

According to a further aspect of the present disclosure, there is provided a usage of the apparatus for cell colony culture *in vitro* in regulating the directed differentiation of stem cell.

It should note that the above-mentioned "stem cell" comprises totipotent stem cell and other kinds of stem cell.

According to another aspect of the present disclosure, there is provided an apparatus for cell colony culture *in vitro.* According to an embodiment of the present disclosure, the apparatus for cell colony culture *in vitro* provided by the present disclosure may comprise a micro-patterned plate, having a plurality of micro-patterned cavities; and an adhesive hydrogel, located on a lower surface of the micro-patterned plate, in which the adhesive hydrogel and the micro-patterned plate are cross-linked and adhered integrally, the adhesive hydrogel constituent the bottom of micro-patterned cavity; the shape and volume of each micro-patterned cavity of the micro-patterned plate determine a physical growth space of cell colony; and the cell seeding density, culture period and culture mode determine a growth pattern (monolayer or multilayer).

According to an embodiment of the present disclosure, the apparatus for cell colony culture *in vitro* may further comprise a hard substrate, located under the adhesive hydrogel, or a multi-well culture plate, located under the adhesive hydrogel and embedded in the micro-patterned plate.

According to an embodiment of the present disclosure, the adhesive hydrogel is prepared using a natural biomaterial and/or synthetic biomaterial, which is cross-linkable to form a gel, the natural biomaterial is at least one selected from a group consisting of gelatin, gelatin derivative, alginate, alginate derivative, agar, matrigel, collagen, proteoglycan, glycoprotein, hyaluronic acid, laminin and fibronectin; the synthetic biomaterial is at least one selected from a group consisting of polypropylene, polystyrene, polyacrylamide, polylactic, poly-hydroxyl acid, polylactic-alkyd copolymer, poly-dimethylsiloxane, polyanhydride, poly-acid-ester, polyamide, polyamino acid, polyacetal, polycyanoacrylate, polyurethane, polypyrrole, polyester, polymethacrylate, polyethylene, polycarbonate and polyepoxyethane.

According to an embodiment of the present disclosure, a material used for the micro-patterned plate is poly(methyl methacrylate). According to another embodiment of the present disclosure, the material used for the adhesive hydrogel consists of gelatin-methyl methacrylate and a photoinitiator Irgacure 2959 (I2959, 2-hydroxy-4-(2-hydroxyethoxy)-2 methyl propiophenon). According to a further embodiment of the present disclosure, the gelatin-methyl methacrylate is a polymer of gelatin and methyl methacrylate, and a ratio between gelatin and methyl methacrylate is 1 g of gelatin against 1 mL of methyl methacrylate. According to a still further embodiment of the present disclosure, the gelatin-methyl methacrylate may be prepared according to following specific steps: adding 1 g of gelatin into 10 mL of DPBS solution, heating at 50°C in a water bath , stirring the solution till the gelatin is totally melted, adding 1 mL of methyl methacrylate slowly at a speed of 0.5 mL/min for 2 hours reaction, taking the mixed solution out of the water bath for cooling down to the room temperature, adding 40 mL of DPBS solution for diluting, dialyzing the diluted solution at 60°C for one week using a dialysis bag having a molecular weight cut-off of 8000-12000 in deionized water, centrifuging for a supernatant, freeze drying the supernatant for one week to form a white fluffy solid having a foamy shape, storing at -80°C.

According to an embodiment of the present disclosure, the adhesive hydrogel is prepared by the following steps: (1) dissolving gelatin-methyl methacrylate in DMEM medium, to obtain a gelatin-methyl methacrylate solution , wherein a content of gelatin-methyl methacrylate in the gelatin-methyl methacrylate solution is 5 g of gelatin- methyl methacrylate per 100 mL of the gelatin-methyl methacrylate solution; (2) dissolving the photoinitiator 2-hydroxy-4-(2-hydroxyethoxy)-2 methyl propiophenon in absolute ethanol, to obtain a photoinitiator solution, wherein a content of 2-hydroxy-4-(2-hydroxyethoxy)-2 methyl propiophenon in the photoinitiator solution is 10 g of 2-hydroxy-4-(2-hydroxyethoxy)-2 methyl propiophenon per 100 mL of the photoinitiator solution; 3) mixing the photoinitiator solution and the gelatin-methyl methacrylate solution at a volume ratio of 1:20.

According to an embodiment of the present disclosure, the DMEM medium may be commercially obtained, such as the product with a catalog number of 11965092 from Invitrogen Company.

According to an embodiment of the present disclosure, when preparing the adhesive hydrogel, extracellular matrix component may be further coated on an upper surface of the adhesive hydrogel as required. According to another embodiment of the present disclosure, the extracellular matrix component comprises collagen, gelatin, matrigel, proteoglycan, glycoprotein, hyaluronic acid, laminin and fibronectin. According to a further embodiment of the present disclosure, the adhesive hydrogel may also be fitted with growth factor and chemical signal molecule. According to a still another embodiment of the present disclosure, the adhesive hydrogel has a thickness of 1-100 µm.

According to embodiments of the present disclosure, the apparatus for cell colony culture *in vitro* may be constructed according to following steps: placing the micro-patterned plate into the multi-well culture plate, evenly injecting a solution of the adhesive hydrogel containing a cross-linking agent along bottom surface of the micro-patterned plate, connecting the micro-patterned plate and the multi-well culture plate as an entirety by means of cross-linked action of the cross-linking agent and adhesive force of the adhesive hydrogel, to form an apparatus for cell colony culture *in vitro* having a construction of micro-patterned plate--substrate adhesive hydrogel--surface of culture dish from top to bottom successively.

According to embodiments of the present disclosure, using the apparatus for cell colony culture *in vitro* provided by the present disclosure, may achieve homogeneous growth of cell colony.

It should note that uses of the apparatus for cell colony culture *in vitro* provided by the present disclosure in regulating self-renewal and/or directed differentiation of totipotent stem cell and other kinds of stem cell are all fallen into the protection scope of the present disclosure.

According to an embodiment of the present disclosure, the totipotent stem cell is human embryonic stem cell; the directed differentiation refers to human embryonic stem cell differentiate into hepatocyte-like cells.

According to another aspect of the present disclosure, there is provided a method for regulating the self-renewal of stem cell. According to embodiments of the present disclosure, the method may comprise culturing the stem cell using the apparatus for cell colony culture *in vitro* above-mentioned.

According to a further aspect of the present disclosure, there is provided a method regulating the directed differentiation of stem cell. According to embodiments of the present disclosure, the method may comprise culturing the stem cell using the apparatus for cell colony culture *in vitro* above-mentioned.

It should note that the above-mentioned "stem cell" may comprise totipotent stem cell and other kinds of stem cell.

According to a still further aspect of the present disclosure, there is provided a method for forming a monolayer micro-pattern of cell. According to embodiments of the present disclosure, the method may comprise culturing the cell using the apparatus for cell colony culture *in vitro* above-mentioned, to obtain the monolayer micro-pattern of cell, in which the cell is at least one selected from stem cell and adult cell.

According to another aspect of the present disclosure, there is provided a method for forming a multilayer micro-cluster of cell. According to embodiments of the present disclosure, the method may comprise culturing the cell using the apparatus for cell colony culture *in vitro* above-mentioned, to obtain the multilayer micro-cluster of cell, in which the cell is at least one selected from stem cell and adult cell.

It should note that the present disclosure has following advantages comparing with the existing research:
1. The apparatus for cell colony culture *in vitro* provided by the present disclosure integrally using micro-patterned plate and adhesive hydrogel, may achieve the regulation to various factors such as shape and size of cell colony, cell monolayer or multilayer growth pattern, chemical strength and chemical component of adhesive substrate, growth factor and chemical signal, co-cultured cell; while the regulations to various factors are independent without mutual interference.
2. The apparatus for cell colony culture *in vitro* provided by the present disclosure may achieve proliferation and induced differentiation of cell *in situ,* which greatly simplifies the operations, and can reduce treatment and damage to cell.
3. The apparatus for cell colony culture *in vitro* provided by the present disclosure may achieve keeping an excellent sterile environment without changing original operating steps and details of cell culture by together using a commercial culture dish, which makes the preparation thereof simple, convenient and easy.
4. The apparatus for cell colony culture *in vitro* provided by the present disclosure may conveniently construct various cell co-culturing environment at the same time by taking full advantage of micro-patterned technique, which provides convenient and practical method for researches of model construction and screening with high-throughput and high-content.

Additional aspects and advantages of embodiments of present disclosure will be given in part in the following descriptions, become apparent in part from the following descriptions, or be learned from the practice of the embodiments of the present disclosure.

### BRIEF DESCRIPTION OF THE DRAWINGS

These and other aspects and advantages of embodiments of the present disclosure will become apparent and more readily appreciated from the following descriptions made with reference to the accompanying drawings, in which:

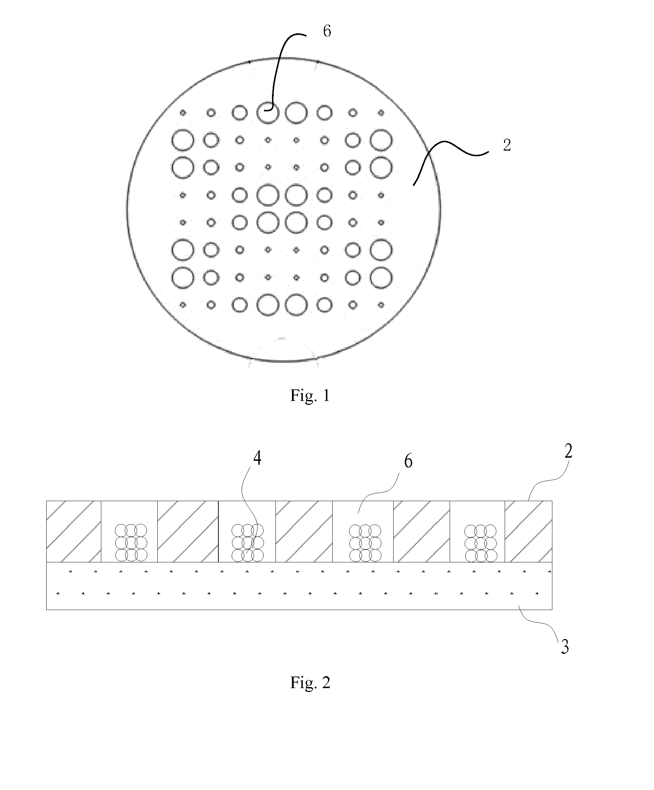

Fig. 1 is a designing schematic diagram of a micro-patterned plate according to an embodiment of the present disclosure, in which four kinds of circles with different sizes of diameter shown in the figure successively represents a growth space for cell culture respectively having a diameter of 300 µm, 500 µm, 1000 µm or 1500 µm according to the order of the diameter from small to large;
Fig. 2 is a structural diagram of an apparatus for cell colony culture *in vitro* according to an embodiment of the present disclosure, in which 2 represents a micro-patterned plate, 3 represents a layer of a substrate hydrogel, 4 represents a cell colony, 6 represents a micro-patterned cavity;

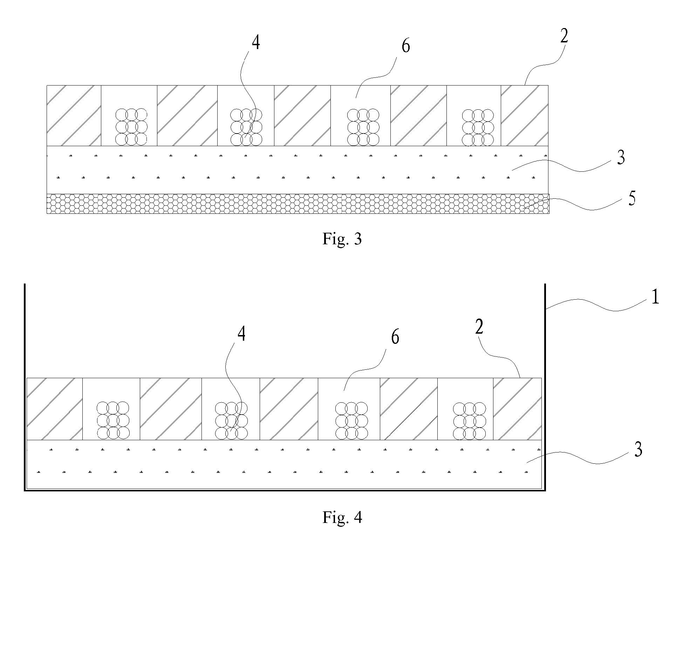

Fig. 3 is a structural diagram of an apparatus for cell colony culture *in vitro* according to another embodiment of the present disclosure, in which 2 represents a micro-patterned plate, 3 represents a layer of a substrate hydrogel, 4 represents a cell colony, 5 represents a hard substrate, 6 represents a micro-patterned cavity;
Fig. 4 is a structural diagram of an apparatus for cell colony culture *in vitro* according to a further embodiment of the present disclosure, in which 1 represents a culture plate, 2 represents a micro-patterned plate, 3 represents a layer of a substrate hydrogel, 4 represents a cell colony, 6 represents a micro-patterned cavity;

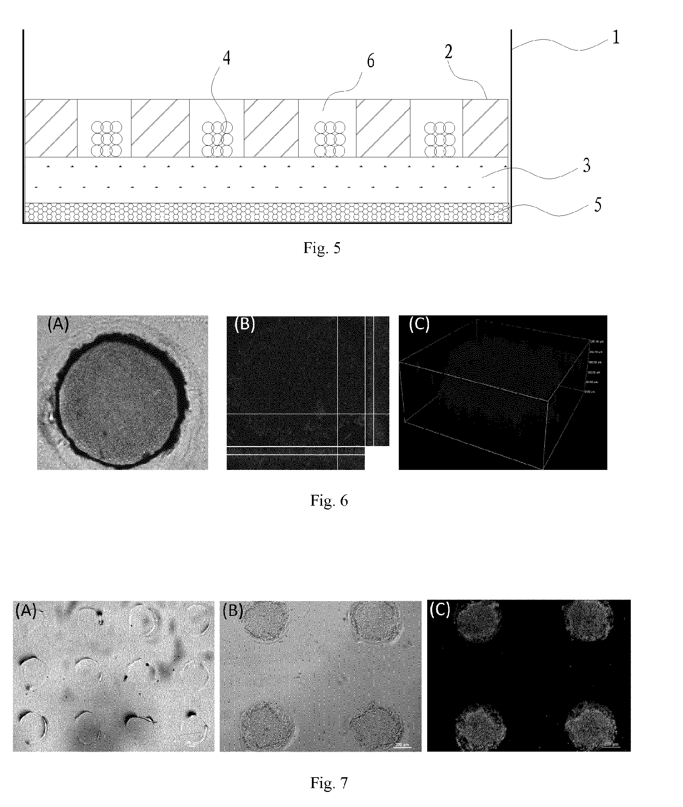

Fig. 5 is a structural diagram of an apparatus for cell colony culture *in vitro* according to a still further embodiment of the present disclosure, in which 1 represents a culture plate, 2 represents a micro-patterned plate, 3 represents a layer of a substrate hydrogel, 4 represents a cell colony, 5 represents a hard substrate, 6 represents a micro-patterned cavity;
Fig. 6 is micro-patterned growth of a monolayer cell in the apparatus for cell colony culture *in vitro* according to an embodiment of the present disclosure, in which (A) shows growth on the micro-patterned hydrogel substrate after disassembling the micro-pattered plate on the top, (B) shows micro-patterned growth of homogeneous monolayer cell formed by mouse embryo fibroblast (NIH/3T3), (C) shows a micro-patterned growth of a monolayer cell detected by fluorescence-substrated LIVE/DEAD cell viability assay, indicating an excellent cell viability (green fluorescence indicates live cells, red fluorescence indicates dead cells);
Fig. 7 is micro-cluster growth of homogeneous multilayer cell formed in the apparatus for cell colony culture *in vitro* according to an embodiment of the present disclosure, in which (A) shows micro-cluster growth of multilayer cell observed under optical microscope, (B) is a side view showing micro-cluster growth of multilayer cell observed under a laser scanning confocal microscope by means of three-dimensional reconstruction method, (C) is an overall figure showing micro-cluster growth of multilayer cell observed under a laser scanning confocal microscope by means of three-dimensional reconstruction method;

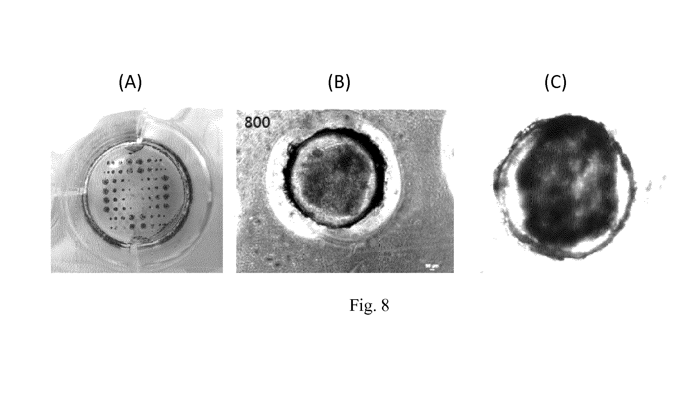

Fig. 8 is micro-cluster growth of hESC multilayer cell formed in the apparatus for cell colony culture *in vitro* according to an embodiment of the present disclosure, in which (A) shows micro-cluster growth of hESC multilayer cell macro-observed by alkaline phosphatase staining, the totipotent stem cell becomes purple red by alkaline phosphatase staining, (B) shows micro-cluster growth of hESC multilayer cell observed by microscope, the shown micro-pattern has a diameter of 800 micrometer, (C) shows micro-cluster growth of hESC multilayer cell observed under microscope by alkaline phosphatase staining, positive staining of purple red indicates the totipotent hESC.

### DETAILED DESCRIPTION

References will be made in detail to embodiments of the present disclosure, examples of the embodiment will be shown in figures, in which the same or similar reference numerals represent same or similar element or the elements having same or similar functions throughout the descriptions. The embodiments described hereafter with reference to figures are explanatory, illustrative, and used to generally understand the present disclosure, which shall not be understood as a restriction to the present disclosure.

In the specification of the present disclosure, it should note that relative terms indicating orientation or orientative relationship such as "upper", "lower", "top", "bottom" and *et al* are substrated on the orientation or orientative relationship shown in the figures, which are just for convenience of describing the present disclosure and simplifying the description, and do not indict or suggest that the indicated apparatus or element must have a particular orientation, and be constructed or operated in a particular orientation, thus shall not be understood as a restriction to the present disclosure.

Unless otherwise stated, the term "multi-", "a plurality of" refers to two or more.

According to an aspect of the present disclosure, there is provided an apparatus for cell colony culture *in vitro.* According to embodiments of the present disclosure, the apparatus for cell colony culture *in vitro* comprises a micro-patterned plate and an adhesive hydrogel. According to embodiments of the present disclosure, the micro-patterned plate has a micro-patterned cavity, which defines a growth space for cell colony, the adhesive hydrogel forms on a lower surface of the micro-patterned plate.

According to embodiments of the present disclosure, using the apparatus for cell colony culture *in vitro* of the present disclosure may achieve a homogeneous growth of cell colony. In particular, according to some embodiments of the present disclosure, the apparatus for cell colony culture *in vitro* of the present disclosure, integrally using the micro-patterned plate and the adhesive hydrogel, may achieve regulations on various factors, such as shape and size of cell colony, mechanical strength and chemical component of adhesive substrate, growth factor and chemical signal, co-cultured cell; while the regulations to various factors are independent without mutual interference; may achieve proliferation and induced differentiation of cell *in situ,* which greatly simplifies the operations, and can reduce treatment and damage to cell; may conveniently construct various cell co-culturing environment at the same time by taking full advantage of micro-patterned technique, which provides convenient and practical method for researches of model construction and screening with high-throughput and high-content.

Reference will be made in detail hereafter to the apparatus for cell colony culture *in vitro* of the present disclosure with figures 1 to 5:

According to embodiment of the present disclosure, the apparatus for cell colony culture *in vitro* may comprise a micro-patterned plate 2 and an adhesive hydrogel 3. According to embodiments of the present disclosure, the micro-patterned plate 2 may having a micro-patterned cavity 6, the adhesive hydrogel 3 may from on a lower surface of the micro-patterned plate 2, in which the micro-patterned cavity 6 defines a growth space for cell colony 4.

According to an embodiment of the present disclosure, the apparatus for cell colony culture *in vitro* of the present disclosure may further comprise: a hard substrate 5, which may locate on a lower surface of the adhesive hydrogel 3. According to embodiments of the present disclosure, the material of the hard substrate 5 is not subjected to special restriction, which may be made by a material such as glass or plastic. According to an embodiment of the present disclosure, the apparatus for cell colony culture *in vitro* of the present disclosure may further comprise: a multi-well culture plate 1, which may locate under the adhesive hydrogel 3 and embed in the micro-patterned plate 2. According to an embodiment of the present disclosure, the adhesive hydrogel 3 may be formed by cross-linking at least one selected from a group consisting of a natural biomaterial and a synthetic biomaterial. According to an embodiment of the present disclosure, the natural biomaterial is at least one selected from a group consisting of gelatin, gelatin derivative, alginate, alginate derivative, agar, matrigel, collagen, proteoglycan, glycoprotein, hyaluronic acid, laminin and fibronectin. According to an embodiment of the present disclosure, the synthetic biomaterial is at least one selected from a group consisting of polypropylene, polystyrene, polyacrylamide, polylactic, poly-hydroxyl acid, polylactic-alkyd copolymer, poly-dimethylsiloxane, polyanhydride poly-acid-ester, polyamide, polyamino acid, polyacetal, polycyanoacrylate, polyurethane, polypyrrole, polyester, polymethacrylate, polyethylene, polycarbonate and polyepoxyethane.

According to an embodiment of the present disclosure, the micro-patterned plate 2 may be formed by poly(methyl methacrylate). According to an embodiment of the present disclosure, the adhesive hydrogel 3 may be formed from gelatin-methyl methacrylate and a photoinitiator, in which the photoinitiator may be 2-hydroxy-4-(2-hydroxyethoxy)-2-methyl propiophenon. According to an embodiment of the present disclosure, the gelatin-methyl methacrylate may be a polymer of gelatin and methyl methacrylate at a ratio of 1 g of gelatin against 1 mL of methyl methacrylate.

According to an embodiment of the present disclosure, the adhesive hydrogel 3 may be prepared by the following steps:
firstly, gelatin-methyl methacrylate is dissolved in DMEM medium, to obtain a gelatin-methyl methacrylate solution, in which a content of gelatin-methyl methacrylate in the gelatin-methyl methacrylate solution is 5 g of gelatin- methyl methacrylate per 100 mL of the gelatin-methyl methacrylate solution;
secondly, the photoinitiator is dissolved in absolute ethanol, to obtain a photoinitiator solution, in which a content of the photoinitiator in the photoinitiator solution is 10 g of the photoinitiator per 100 mL of the photoinitiator solution;
thirdly, the photoinitiator solution and the gelatin-methyl methacrylate solution are mixed at a volume ratio of 1:20;
lastly, gelatin-methyl methacrylate is subjected to cross-linking using ultraviolet ray, to obtain the adhesive hydrogel 3.

According to an embodiment of the present disclosure, the adhesive hydrogel 3 may comprise at least one selected from a group consisting of extracellular matrix component, growth factor and chemical signaling molecule. According to an embodiment of the present disclosure, the extracellular matrix may be coated on an upper surface of the adhesive hydrogel 3. According to an embodiment of the present disclosure, the extracellular matrix component may be at least one selected from a group consisting of collagen, gelatin, matrigel, proteoglycan, glycoprotein, hyaluronic acid, laminin and fibronectin. According to an embodiment of the present disclosure, the adhesive hydrogel 3 may be fitted with the growth factor and the chemical signal molecule.

According to an embodiment of the present disclosure, the adhesive hydrogel 3 has a thickness of 1-100 µm.

According to embodiments of the present disclosure, the adhesive hydrogel 3 may serve as a growth substrate supporting for culturing cell (like totipotent stem cell) or feeder cell (like mouse embryonic fibroblast).

According to another aspect of the present disclosure, there is provided an apparatus for cell colony culture *in vitro.* It should note that following described features and the effects for the apparatus for cell colony culture *in vitro,* duly suitable for the apparatus for cell colony culture *in vitro* above-described.

According to an embodiment of the present disclosure, the apparatus for cell colony culture *in vitro* provided by the present disclosure comprises a micro-patterned plate 2 having a plurality of micro-patterned cavities 6 and an adhesive hydrogel 3 located on a lower surface of the micro-patterned plate 2; in which the adhesive hydrogel 3 and the micro-patterned plate 2 are cross-linked and adhered integrally; the shape and volume of each micro-patterned cavity 6 of the micro-patterned plate 2 determine a physical growth space of cell colony 4.

According to an embodiment of the present disclosure, as required, the apparatus for cell colony culture *in vitro* may further comprise a hard substrate 5 located under the adhesive hydrogel 3, or a multi-well culture plate 1 located under the adhesive hydrogel 3 and embedded in the micro-patterned plate 2.

According to an embodiment of the present disclosure, the micro-patterned plate 2 upper located may be selected to disassemble or used fixedly, to adapt to various post-uses such as cell monolayer growth, multilayer growth, cell co-culture or totipotent stem cell differentiation, and *et al.*

According to an embodiment of the present disclosure, the micro-patterned plate 2 may be obtained by micro-patterned method well-known to the art, for example, laser cutting and engraving, molding by micro patterned mould and *et al;* and, the shape and size of the growth space for cell colony 4 and the integral micro-patterned plate 2 may be adjusted by combining computer-aided designing and manufacturing technology. In particular, according to some embodiments of the present disclosure, using the computer-aided designing and manufacturing technology may conveniently achieve various shape and size of the micro-patterned cavity 6, for example, the shape may be circle, square, rectangle, diamond, trapezoid, various irregular shapes and *et al,* respectively having a different size of 10 µm to 2 cm, to determine a physical space for cell colony 4 growth; the integral size and shape of the micro-patterned plate 2 are usually determined according to the size of the used culture plate 1, the shape is usually regular circle, a diameter thereof is equal to or slightly less than that of the culture plate 1, the a micro-patterned plate 2 with a desired size and shape may be conveniently prepared by computer-aided designing and manufacturing technology; the preferred thickness of the micro-patterned plate 2 is for material saving and convenient use, which is usually different in a range of 100 µm to 2 cm.

According to an embodiment of the present disclosure, the adhesive hydrogel 3 is a growth substrate supporting for culturing cell (like totipotent stem cell) or feeder cell (like mouse embryonic fibroblast). According to an embodiment of the present disclosure, the adhesive hydrogel 3 is prepared using a natural biomaterial and/or synthetic biomaterial, which is cross-linkable to form a gel, in which the natural biomaterial is at least one selected from a group consisting of gelatin, gelatin derivative, alginate, alginate derivative, agar, matrigel, collagen, proteoglycan, glycoprotein, hyaluronic acid, laminin and fibronectin, the synthetic biomaterial is at least one selected from a group consisting of polypropylene, polystyrene, polyacrylamide, polylactic, poly-hydroxyl acid, polylactic-alkyd copolymer, poly-dimethylsiloxane, polyanhydride, poly-acid-ester, polyamide, polyamino acid, polyacetal, polycyanoacrylate, polyurethane, polypyrrole, polyester, polymethacrylate, polyethylene, polycarbonate and polyepoxyethane. According to an embodiment of the present disclosure, the material forming the substrate (the adhesive hydrogel 3 lower located) may be one or more mixtures of the above-mentioned materials. The type and usage amount of the cross-linking agent depend on the type and amount of the substrate biological material, under a premise of sufficient cross-linkage.

According to an embodiment of the present disclosure, material used for the micro-patterned plate 2 may be poly(methyl methacrylate); material used for the adhesive hydrogel 3 specifically may consists of gelatin-methyl methacrylate and a photoinitiator Irgacure 2959 (I2959, 2-hydroxy-4-(2-hydroxyethoxy)-2 methyl propiophenon); the gelatin-methyl methacrylate is a polymer of gelatin and methyl methacrylate, and a ratio between gelatin and methyl methacrylate is 1 g of gelatin against 1 mL of methyl methacrylate. According to an embodiment of the present disclosure, the gelatin-methyl methacrylate may be prepared according to following specific steps: adding 1 g of gelatin into 10 mL of DPBS solution, heating at 50°C in a water bath , stirring the solution till the gelatin is totally melted, adding 1 mL of methyl methacrylate slowly at a speed of 0.5 mL/min for 2 hours reaction, taking the mixed solution out of the water bath for cooling down to the room temperature, adding 40 mL of DPBS solution for diluting, dialyzing the diluted solution at 60°C for one week using a dialysis bag having a molecular weight cut-off of 8000-12000 in deionized water, centrifuging for a supernatant, freeze drying the supernatant for one week to form a white fluffy solid having a foamy shape, storing at -80°C.

According to an embodiment of the present disclosure, the adhesive hydrogel 3 may be prepared by the specifically following specific steps:
(1) dissolving gelatin-methyl methacrylate in DMEM medium, to obtain a gelatin-methyl methacrylate solution , wherein a content of gelatin-methyl methacrylate in the gelatin-methyl methacrylate solution is 5 g of gelatin- methyl methacrylate per 100 mL of the gelatin-methyl methacrylate solution; (2) dissolving the photoinitiator 2-hydroxy-4-(2-hydroxyethoxy)-2 methyl propiophenon in absolute ethanol, to obtain a photoinitiator solution, wherein a content of 2-hydroxy-4-(2-hydroxyethoxy)-2 methyl propiophenon in the photoinitiator solution is 10 g of 2-hydroxy-4-(2-hydroxyethoxy)-2 methyl propiophenon per 100 mL of the photoinitiator solution;
3) mixing the photoinitiator solution and the gelatin-methyl methacrylate solution at a volume ratio of 1:20.

According to an embodiment of the present disclosure, the DMEM medium may be commercially obtained, such as the product with a catalog number of 11965092 from Invitrogen Company.

According to an embodiment of the present disclosure, as required, extracellular matrix component may be further coated on an upper surface of the adhesive hydrogel 3; the extracellular matrix component comprises collagen, gelatin, matrigel, proteoglycan, glycoprotein, hyaluronic acid, laminin and fibronectin; the adhesive hydrogel 3 may also be fitted with growth factor and chemical signal molecule.

According to an embodiment of the present disclosure, usage amount of the adhesive hydrogel 3 material depends on usable area of the culture plate 1, under a premise of even coverage on surface of the culture plate 1 or the hard substrate 5, which is usually different in a range of 1 µl to 10 mL.

According to an embodiment of the present disclosure, the method of constructing the apparatus for cell colony culture *in vitro* includes but not limited to following 4 kinds:
1) a micro-patterned plate 2, after being designed and prepared with a desired size and shape, is placed into a multi-well culture plate 1; a substrate biological material is subjected to cross-linking after being evenly injected into the culture plate 1 along the bottom surface thereof; by means of cross-linked action of the cross-linking agent and adhesive force of the adhesive hydrogel 3, the micro-patterned plate 2 and the multi-well culture plate 3 are connected as an entirety; extra cross-linking agent and bubble are removed after sufficient cross-linking; the obtained apparatus for cell colony culture *in vitro* is immediately used or preserved at low temperature for use.
2) a substrate biological material is subjected to cross-linking firstly after being evenly injected into culture plate 1 to form a substrate hydrogel 3, then a micro-patterned plate 2 and the substrate hydrogel 3 are connected as an entirety by means of stress; extra cross-linking agent and bubble are removed after sufficient cross-linking; the obtained apparatus for cell colony culture *in vitro* is immediately used or preserved at low temperature for use.
3) a hard substrate 5 (such as glass sheet and plastic sheet) is processed to have an integral size consistent with that of a micro-patterned plate 2; a substrate hydrogel 3 is formed on the hard substrate 5; then the micro-patterned plate 2 is connected to the substrate hydrogel 3 to form an entirety by means of stress, the formed entirety has a three-layer integral construction of hard substrate --hydrogel--plate from top to bottom successively; extra cross-linking agent and bubble are removed after sufficient cross-linking; the obtained apparatus for cell colony culture *in vitro* is immediately used or preserved at low temperature for use.
4) a hard substrate 5 with a slice shape is aligned or adhered from bottom to top with a micro-patterned plate 2; a substrate biological material is subjected to cross-linking after being evenly injected along an interval between the hard substrate 5 and the micro-patterned plate 2, to form a three integral construction of hard substrate--hydrogel--template from top to bottom successively; the obtained three integral structure is embedded into a culture plate 1, extra cross-linking agent and bubble are removed after sufficient cross-linking; the obtained apparatus for cell colony culture *in vitro* is immediately used or preserved at low temperature for use.

According to an embodiment of the present disclosure, the apparatus for cell colony culture *in vitro* provided by the present disclosure may achieve homogeneous growth of cell colony, which may be used in regulating self-renewal and directed differentiation of totipotent stem cell and other kinds of stem cell, mainly comprising a regulation to physical factor (such as colony shape, size, substrate mechanical strength and *et al*), chemical factor (such as substrate component, growth factor, chemical signal and *et al*) and biological factor (such as co-cultured cell and *et al*).

According to an embodiment of the present disclosure, the implementation method of regulating the physical factor (such as colony shape, size, substrate mechanical strength and *et al*) of the present disclosure is shown below: cutting off template material to form a micro-patterned cavity 6, to construct a physical space for totipotent stem cell colony 4, by which the shape and size of colony depends on shape and size of the micro-patterned cavity 6 in the plate. By computer-aided designing and manufacturing technology may conveniently achieve various shape and size of the micro-patterned cavity 6, and cutting off to form the micro-patterned cavity 6 with different physical property in one template may achieve a comparison of shape, size and distribution of different colony under one same culture condition, which is of great significance in high-throughput research and screening. The shape of the micro-patterned cavity 6 is circle, square, rectangle, diamond, trapezoid, various irregular shapes and *et al,* respectively having a different size of 10 µm to 2 cm.

According to an embodiment of the present disclosure, substrate mechanical stress is determined by kinds, usage amount and cross-linking degree of the material of the substrate hydrogel 3 (material of adhesive hydrogel 3). In general, the more usage amount of the substrate hydrogel 3, the higher degree of cross-linking, then the more intense of the mechanical stress. In particular, according to an embodiment of the present disclosure, the mechanical stress of the substrate may be meddled during using the apparatus for cell colony culture *in vitro* by a process of artificial regulation or self-regulation and *et al.* The artificial regulation to the mechanical stress of the substrate is mainly achieved by twice or multiple cross-linking of the substrate hydrogel material, for example the substrate hydrogel material, formed by gelatin-methyl methacrylate, may conveniently achieve twice or multiple cross-linking *in situ* at a desired time, by physical cross-linking method (such as ultraviolet light, blue light and *et al*) and chemical cross-linking method (such as Genipin, glutaraldehyde and *et al*), a result thereof commonly is the improvement of chemical stress of substrate hydrogel material. The self-regulation to mechanical stress of the substrate hydrogel material is mainly achieved by interaction of cell-material and degradation of material during cell culture, a result thereof commonly is the decrease of mechanical stress of the substrate hydrogel material. According to an embodiment, a combination of the self-regulation and the artificial regulation may achieve multiple time regulation and control to the mechanical stress of the substrate hydrogel material *in situ* at any time, to adapt to a requirement of self-renewal and directed differentiation of totipotent stem cell, for example early in the initial period of totipotent stem cell, relative lower chemical stress of the substrate is beneficial to maintenance of self-renewal and totipotency of the stem cell; after the totipotent stem cell is subjected to an inducing culture for directed differentiation after proliferating and forming into a homogeneous colony (such as differentiating towards to hepatocyte-like, cardiomyocyte, pancreatic cell and *et al,* then relative higher chemical stress of the substrate is beneficial to directed differentiation of stem cell by this time. According to an embodiment of the present disclosure, using the apparatus for cell colony culture *in vitro* and the method of regulation thereof may achieve self-renewal and directed differentiation inducing culture of the totipotent stem cell *in situ.* According to an embodiment of the present disclosure, usage amount of the above-mentioned substrate hydrogel material depends on usable area of the culture plate 1, under a premise of even coverage on surface of the culture plate 1 or the hard substrate 5, which is usually different in a range of 1 µl to 10 mL, type and usage amount of the cross-linking agent depend on type and usage amount of the substrate biological material, under a premise of sufficient cross-linkage.

According to an embodiment of the present disclosure, specific method of regulating chemical factors such as substrate component , growth factor, chemical signal molecule and *et al* according to the present disclosure is shown as below: due to different biological and physicochemical property of upper micro-patterned template 2 and lower adhesive hydrogel 3, different vertical height, as well as subsequent operations such as percussion suction, a surface of the substrate hydrogel material usually is the surface directly adhered with growing totipotent stem cell or feeder cell, coating various biological materials on surface of the substrate hydrogel may achieve substrate component modification and regulation, for example coating with various extracellular matrix components such as collagen, gelatin, matrigel, proteoglycan, glycoprotein, hyaluronic acid, laminin and fibronectin, to improve cell adhesion, to better maintain self-renewal of stem cell, or to promote directed differentiation of totipotent stem cell. According to an embodiment of the present disclosure, the coating material herein is not limited to the above type of material; specific type and dosage of use depend on cell type and culture purpose.

According to an embodiment of the present disclosure, the present disclosure may conveniently achieve compounding and slow-releasing of various growth factors and chemical signal molecules in the apparatus for cell colony culture *in vitro.* According to embodiments of the present disclosure, using factor immobilization to embed the growth factor and the chemical signal molecule into the substrate hydrogel 3, may achieve a bionic, efficient and controllable slow-releasing of the various factors, so as to improve acting efficiency and acting period of the factor, which provides a convenient and useful model for in-vitro simulating in-vivo development, and has significant economic benefit. At the same time, according to another embodiment of the present disclosure, growth factor and chemical signal molecule may also participate in cell culture process in the form of soluble molecule, which may conveniently add factors scheduled-controllably, to adapt to a requirement of scheduled inducing differentiation culture. According to an embodiment of the present disclosure, selection of the growth factor and chemical signal molecule herein depends on cell type and culture purpose.

According to embodiments of the present disclosure, the present disclosure may suitable for various usages such as proliferative culture, inducing differential culture of totipotent stem cell and *et al,* the convenient culturing approach is a significant guarantee to achieve the above functions. According to some embodiments of the present disclosure, specific method of the present disclosure in regulating biological factors such as co-culture cell and et al includes but not limited following two kinds:
1) co-culturing with feeder cell for maintaining self-renewal of totipotent stem cell. According to an embodiment of the present disclosure, feeder cell may be firstly selectively seeded in the apparatus for cell colony culture *in vitro,* to adhere onto the substrate hydrogel surface of the micro-patterned cavity 6 or the micro-patterned template 2; then totipotent stem cell is seeded in the apparatus for cell colony culture *in vitro,* form a physiological environment for co-culturing with the feeder cell, which may be benefit to the maintenance of self-renewal and totipotency of the totipotent stem cell. According to an embodiment of the present disclosure, selection and seeding density of the feeder cell herein are well-known in the art, specifically depending on totipotent stem cell type.
2) auxiliary cell co-culturing for promoting directed differentiation of totipotent stem cell. According to an embodiment of the present disclosure, the totipotent stem cell may be subjected to inducing culture of stem cell directed differentiation in situ after forming a homogeneous colony in the apparatus for cell colony culture *in vitro;* by this time the auxiliary cell co-culture may be selectively conducted, method of co-culturing comprises retaining micro-patterned plate and disassembling micro-patterned plate.

According to an embodiment of the present disclosure, an auxiliary cell may be directly seeded in the apparatus for cell colony culture *in vitro* in the case of reserving template, by this time, the auxiliary cell preferably adheres onto a non-cavity surface of a micro-patterned plate 2, to form a non-contacted co-culturing environment for the auxiliary cell and totipotent stem cell colony; in addition, the upper micro-patterned template 2 may be disassembled after forming a homogeneous colony of totipotent stem cell, while retaining totipotent stem cell colony 4, substrate hydrogel 3 and/or hard substrate t, by this time the seeded auxiliary cell preferably attached onto a blank surface of the substrate hydrogel 3 which is unoccupied by totipotent stem cell colony 4, to form a contacted co-culturing environment for the auxiliary cell and totipotent stem cell colony. According to embodiments of the present disclosure, selections of the above two ways depend on factors such as substrate hydrogel material type, inducing differential direction of totipotent stem cell and auxiliary cell type, *et al.* Type and seeding density of the auxiliary cell are well-known in the art, which mainly depends on the number of totipotent stem cell colony, cell density and inducing differential direction of stem cell, *et al.*

According to another aspect of the present disclosure, there is provided a usage of the apparatus for cell colony culture *in vitro* above-mentioned in regulating the self-renewal of stem cell.

According to a further aspect of the present disclosure, there is provided a usage of the apparatus for cell colony culture *in vitro* above-mentioned in regulating the directed differentiation of stem cell.

According to a still further aspect of the present disclosure, there is provided a method for regulating the self-renewal of stem cell. According to embodiments of the present disclosure, the method may comprise: culturing the stem cell using the apparatus for cell colony culture *in vitro* above-mentioned.

According to a more aspect of the present disclosure, there is provided a method for regulating the directed differentiation of stem cell and other kinds of stem cell. According to embodiments of the present disclosure, the method may comprise: culturing the stem cell using the apparatus for cell colony culture *in vitro* above-mentioned.

It should note that type of the above described "stem cell" is not subjected to special restriction, which may include totipotent stem cell and other kinds of stem cell.

According to another aspect of the present disclosure, there is provided a method for forming a monolayer micro-pattern of cell. According to embodiments of the present disclosure, the method may comprise: culturing the stem cell using the apparatus for cell colony culture *in vitro* above-mentioned, to obtain the monolayer micro-pattern of cell, in which the cell is at least one selected from stem cell and adult cell.

According to a further aspect of the present disclosure, there is provided a method for forming a multilayer micro-cluster of cell. According to embodiments of the present disclosure, the method may comprise: culturing the stem cell using the apparatus for cell colony culture *in vitro* above-mentioned, to obtain the multilayer micro-cluster of cell, in which the cell is at least one selected from stem cell and adult cell.

It should note that the apparatus for cell colony culture *in vitro* of the present disclosure is particularly suitable for culturing totipotent stem cell, and may be used to culture other kinds of cell.

Reference will be made in detail to specific examples of the present disclosure. It should be noted that theses following examples are explanatory, and cannot be construed to limit the scope of the present disclosure. If not specified, the used experimental method in the following examples is a conventional method well-known to people skilled in the art. All reagents and products used in the following examples are commercially available, unless a specific statement otherwise.

### Example 1 Preparation of apparatus for cell colony culture in vitro

Referring to Fig. 1 and Fig. 4, the apparatus for cell colony culture *in vitro* was prepared according to following steps:

### 1. Preparation of micro-patterned template

An upper micro-patterned template 2 was prepared by laser cutting. Specific operations were shown below: a polymethyl methacrylate sheet (Technology of Zunbao) having a thickness of 0.5 cm was cut to form an upper micro-pattered template 2 using a Rayjet laser engraving machine. As shown in Fig.1, the micro-pattered template was designed by an AutoCAD software: the designed template had a shape of circle, having a diameter size of 22.1 mm; 8×8 cavities having a diameter of 300 µm, 500 µm, 1000 µm or 1500 µm evenly distributed in a square shape in center of the designed template were served as a culture space for micro-patterned cell (like totipotent stem cell); the distance between two centers of adjacent cavity is 1500 µm. The main processing parameters of laser engraving machine are: cutting power 100%, cutting frequency 2, and cutting linear velocity 10%.

The processed and molded polymethyl methacrylate template was rinsed twice using deionized water, ultrasonic cleaned for 30 min, and then subjected to heat treatment to improve flatness: on a smooth and clean heating template, with a setting temperature of 90°C, the polymethyl methacrylate template was subjected to heating treatment under a stressed state.

The polymethyl methacrylate template after heating treatment was rinsed by immersing in sterile distilled water for 30 min, and subjected to UV-sterilization by double-side irradiation in a safety cabinet after wiped with a sterile paper, to obtain the micro-patterned template 2 for use.

### 2. Preparation of the apparatus for cell colony culture in vitro

A photoinitiator solution having a concentration of 0.1 g/mL was prepared by dissolving the photoinitiator (I2959, 2-hydroxy-4-(2-hydroxyethoxy)-2 methyl propiophenon, 106797-53-9, Insight High Technology Co., Ltd, China) in absolute ethanol, then filtering with a 0.2 µm filter membrane to remove bacterial, dispersing into a 1 mL tube, and was preserved at a low temperature of 4°C avoiding from light.

The gelatin-methyl methacrylate was prepared according to following steps: 1 g of gelatin was added into 10 mL of DPBS solution, then heated at 50°C in a water bath , and stirred the solution till the gelatin was totally melted; 1 mL of methyl methacrylate was slowly added at a speed of 0.5 mL/min for 2 hours reaction; the mixed solution was taken out of the water bath for cooling down to the room temperature; then 40 mL of DPBS solution was added for diluting, and then the diluted solution was dialyzed at 60°C for one week using a dialysis bag having a molecular weight cut-off of 8000-12000 in deionized water; the dialyzed solution was centrifuged and the obtained supernatant was freeze dried for one week to form a white fluffy solid having a foamy shape; then the obtained gelatin-methyl methacrylate was stored at -80°C.

The above obtained gelatin-methyl methacrylate was dissolved in DMEM medium (11965092, Invitrogen, USA), and subjected to a hydrotropy treatment at 60°C for 3 hours, to form a gelatin-methyl methacrylate solution having a concentration of 0.05 g/mL. The gelatin-methyl methacrylate solution was filtered with a 0.2 µm filter membrane to remove bacterial, and then was dispersed into a 1 mL tube for use.

The above prepared photoinitiator solution having a concentration of 0.1 g/mL and the gelatin-methyl methacrylate solution having a concentration of 0.05 g/mL were evenly mixed at a volume ratio of 1:20 when using, and preserved avoiding from light; subsequent relative operations were all conducted under a dark condition.

In accordance with sterile operation specification in safety cabinet, the above obtained micro-patterned plate 2 was embedded into a culture well of a 12-well plate 1 (Corning Company) at a ratio of 1:1, 70 µL of the sterile gelatin-methyl methacrylate/photoinitiator solution was slowly injected into the micro-patterned plate 2 along a bottom surface thereof under a dark condition, by this time a layer of even substrate biological material was formed between the substrate bottom surface and culture plate surface. The gelatin-methyl methacrylate solution was cross-linked using ultraviolet light having a power of about 5.4 mW/cm² and an acting time of 40 seconds, to form a substrate hydrogel 3 (lower adhesive hydrogel 3) having a thickness of 1 to 100 µm. By this time under a combined effect of cross-linked action of the cross-linking agent and adhesive force of the gelatin-methyl methacrylate hydrogel, the apparatus for cell colony culture *in vitro* having a construction of micro-patterned template-substrate hydrogel-culture plate surface from top to bottom successively (shown as Fig. 4). The apparatus for cell colony culture *in vitro* was rinsed twice using 1 mL DMEM solution per culture well, which was immediately used or sterile preserved at low temperature.

### Example 2 Formation of a multilayer micro-cluster using the apparatus for cell colony culture in vitro prepared in Example 1 (taking NIH/3T3 cell as an example)

The present disclosure was taken NIH/3T3 cell as an example, the multilayer micro-cluster was formed using the apparatus for cell colony culture *in vitro* prepared in Example 1, according to following steps:
A conventional cultured NIH/3T3 cell was digested with 0.25 % trypsin in a culture flask having a culture area of 75 cm²; the digested NIH/3T3 cell was collected after centrifuging and then seeded into the apparatus for cell colony culture *in vitro* prepared in Example 1 with a cell density of 4 × 10⁶ cells/mL; each culturing well contained 1 mL of cell suspension; after 24 hours, an upper film was removed from the micro-patterned template using a forceps; A result was shown in Fig. 6. As can be seen from Fig. 6, a micro-patterned homogeneous cell cluster having a construction of three cell layers was formed. Hoechst nuclear staining showed homogeneity of the micro-cluster and distribution of the multi-layer cell.

### Example 3 Culture of human embryonic stem cell using the apparatus for cell colony culture in vitro prepared in Example 1

### 1. proliferative culture of human embryonic stem cell

MEF medium: each 250 mL of MEF medium contained 255 mL of DMEM medium (11965092, Invitrogen), 25 mL of fetal bovine serum (10099141, Invitrogen), 2.5 mL of Glutamax I (35050061, Invitrogen), 2.5 mL of MEM non-essential amino acid (MEM NEAA) (11140050, Invitrogen), 2.5 mL of penicillin/streptomycin (Pen/strep) (100X(15140122, Invitrogen)).

human embryonic stem cell medium: each 250 mL of human embryonic stem cell medium contained 200 mL of knock out DMEM (10829018, Invitrogen), 50 mL of knockout serum replacer (10828028, Invitrogen), 2.5 mL of Glutamax I (35050061, Invitrogen), 2.5 mL of MEM non-essential amino acid (MEM NEAA) (11140050, Invitrogen), 2.5 mL of penicillin/streptomycin (Pen/strep) (100X(15140122, Invitrogen)), 0.08 mL of human basic fibroblast growth factor having a concentration of 25 µg/mL (hbFGF) (233-FB-025, R&D).

### 1.1 obtaining of mouse embryonic fibroblast (MEF)

The mouse embryonic fibroblast (MEF) was obtained according to specific steps shown below:
(1) subjecting mouse to a treatment with pregnant mare serum gonactotropin (HOR-272, Shanghai Gao Chang Medical technology Co., Ltd), to synchronize estrus and superovulation.
(2) mating female mouse and male mouse at a ratio of 2:1 overnight.
(3) the next day, selecting the female mouse with pessary (i.e. a jelly having a color of milk white or faint yellow, by which can determine that the mouse was pregnant). At this time, the time of mouse being pregnant was recorded as 0.5 d.
(4) selecting pregnant female mouse of 12.5 to 14.5 gestation period as experimental material below.
(5) the pregnant female mouse selected in step (4) was sacrificed by Cervical dislocation method, and dissected on an ultra-clean bench after sterilized in a beaker containing 75% ethanol. The skin of abdomen was cut open firstly to expose uterus, after changing another set of surgical instruments, the near-end of cervix thereof was lifted up using an ophthalmic forceps, to isolate mesometrium. After cutting uterine horn, the uterus was taken out to place in a culture plate containing PBS.
(6) The fetal mouse was taken out and rinsed several times using PBS. After the head, tail, viscera and limbs were removed, the only-reserved trunk was sufficiently rinsed again in PBS to remove red blood cell.
(7) The above trunk section was put into a new culture plating containing a little of PBS, and then cut into tissue pieces having a volume less than 1 mm³ using an ophthalmic forceps.
(8) The above tissue pieces were added with 2 to 4 mL of 0.25% trypsin for 10 to 15 minutes digestion in an incubator; then an equal volume of MEF medium to terminate the digestion, the digested cell was dispersed by means of pipetting, to obtain cell suspension.
(9) The obtained cell suspension was centrifuged at 1500 rpm for 4 minutes. After removing supernatant, red blood cell lysis buffer (3 to 5 fold volume of cell volume) was added into the cell pellet. After slightly pipetting for 1 minute, supernatant was removed by centrifuging. If red blood cell still existed, the above step of lysing red blood cell should be repeated.
(10) After centrifuging to remove supernatant, the cell pellet was re-suspended. Then the cell re-suspension was filtered for collecting filtrate using a screen having a sieve mesh of 200. Then the filtrate was centrifuged to remove supernatant again.
(11) The obtained cell pellet obtained in step (10) using MEF medium was rinsed, after centrifuging to remove supernatant once more, the cell pellet obtained in step (11) was re-suspended and seeded in culture plate. The culture plate seeded with cells was incubated in a constant-temperature incubator with a condition of 37°C, 5% CO₂ and saturated humidity.
(12) The culturing medium was changed at the second day of cell culture, then the culturing medium was changed every 2 days. After 2 to 4 days, MEF with almost-full confluence could be subjected to subculture at a ratio of 1:3.
(13) The cultured cell was subjected to inactivation after collected by X-ray, with a dosage of 70 Gy.

Therefore, the mouse embryonic fibroblast was obtained.

### 1.2 formation of a monolayer MEF micro-pattern using the apparatus for cell colony culture in vitro

The above inactive MEF (hereinafter referred to as In-MEF) was evenly seeded with a cell density of 2.5 × 10⁴ cell/cm² into the apparatus for cell colony culture *in vitro* constructed in Example 1. If an apparatus for cell colony culture *in vitro* sterile preserved at low temperature was used, the used apparatus should be rinsed twice using PBS and incubated at 37°C for 10 minutes before using. After the In-MEF was seeded into the apparatus for cell colony culture *in vitro* and placed in an incubator for 24h standing, an upper film was removed from the micro-patterned plate using a forceps. 1 mL of MEF medium was added into the culture plate per well along well wall for rinsing twice, to remove cell on plate surface and keep the In-MEF in the micro-patterned cavity. 1.5 mL of MEF medium was added into the culture plate per well along well wall for culturing the In-MEF in the incubator, the result was shown in Fig. 7. As can be seen from Fig. 7, the monolayer MEF micro-patter was formed, and an excellent cell viability was indicated by fluorescence-substrated LIVE/DEAD cell viability assay.

### 1.3 formation of MEF-hESC co-cultured micro-pattern

After 24 hours, the culturing MEF medium was removed from the apparatus for cell colony culture *in vitro.* The human embryonic stem cell H9 (Wicell) having a confluence of 60 to 70 percentage conventionally-cultured in a six-well culture plate was evenly passage into the apparatus for cell colony culture *in vitro;* the human embryonic stem cell H9 cultured in one well of the six-well culture plate was passage into one well of the apparatus for cell colony culture *in vitro* containing the above described In-MEF, *i.e.* the human embryonic stem cell H9 was seeded with a volume of 3:1. Then, the apparatus for cell colony culture *in vitro* was placed into the incubator with a condition of 5% CO₂, and 37°C for 10 minutes; the supernatant of medium was removed by slightly inclining the apparatus and suction, to remove the human embryonic stem cell H9 on the template surface and keep the human embryonic stem cell H9 in the micro-patterned cavity. Then, 1.5 mL of medium for culturing human embryonic stem cell H9 was added per well along well wall, and the apparatus for cell colony culture *in vitro* was then placed into the incubator with a condition of 5% CO₂, and 37°C for co-culturing MEF and human embryonic stem cell H9. The co-culturing medium was changed every day along with observation; and biological characteristics of human embryonic stem cell H9 were identified using methods such as alkaline phosphatase activity assay, expression examination of specific surface antigen (like SSEA-3, SSEA-4, TRA-1-60), detection of nuclear transcription factors (like OCT4), and *et al.*

As can be seen from the results, after co-cultured for 3 to 4 days in the apparatus for cell colony culture *in vitro* which was prepared in Example 1, human embryonic stem cell H9 formed a consistent cavity shape with that of the micro-patterned template, which has 8×8 homogeneous colonies having a diameter of 300 µm, 500 µm, 1000 µm or 1500 µm, with excellent biological characteristics of each cell colony.

The above methods of culturing and passaging MEF, conventionally culturing, passaging and assaying human embryonic stem cell H9, were well-known in the art, current classical operation standards was preferably used.

### 2. directed inducing differentiation of human embryonic stem cell in situ

### 1) programmed directed inducing differentiation of human embryonic stem cell in situ

In the above step 1, after co-cultured for about 3 to 4 days, the cultured human embryonic stem cell H9 could be observed, 8×8 homogeneous human embryonic stem cell H9 colonies having a diameter of 300 µm, 500 µm, 1000 µm or 1500 µm were formed in the micro-patterned cavities (the biological characteristics were identified being excellent). Then, the obtained human embryonic stem cell H9 was used to inducing differentiation culture of human embryonic stem cell H9 *in situ* in the apparatus for cell colony culture *in vitro* prepared in Example 1. The cell was continuously differentiating while proliferating, to form a multilayer micro-cluster of cell. The programmed inducing differentiation of hepatocyte-like was taken as an example here with specific steps shown below:
Taken the in vivo liver development as a guidance, the programmed inducing differentiation was divided into successive stages of definitive endoderm induction, hepatic progenitor cell specialized induction, hepatocyte ripening induction and *et al.* The inducing medium for each stage was prepared based on following components, the formula of the inducing medium were: 50% Iscove's modified Dulbecco's medium (IMDM) and 50% F12 NUT-MIX, along with 7 µg/ml insulin, 15 µg/mL transferrin, 450 µmol/L thioglycerol and 5mg/mL bovine serum albumin (BSA). The initial time for inducing was recorded as the first day, and the method of inducing differentiation was: culturing stem cell using a medium having an added component of 10 ng/mL activin and 12 ng/mL human fibroblast growth factor 2 (FGF2) during the first to second day, and changing the cultured medium every day; inducing human embryonic stem cell H9 to differentiate towards endoderm cell using a medium having an added component of 100 ng/mL activin, 20 ng/mL FGF2, 10 ng/mL bone morphogenetic protein 4 (BMP4) and 10 µmol/L LY294003 (a PI3K inhibitor) during the third to fifth day, and changing the inducing medium every day; inducing a directed differentiation towards definitive endoderm using a medium having an added component of 50 ng/mL human fibroblast growth factor 10 (FGF10) during the sixth to eighth day, and changing the inducing medium every day; inducing a directed differentiation towards hepatic progenitor cell using a medium having an added component of 50 ng/mL FGF10, 10⁻⁷ mol/L retinoic acid and 10 µmol/L SB431542 (inhibitor of TGF-Smads signal pathway) during the ninth to tenth day, and changing the inducing medium every day; inducing a directed differentiation towards hepatocyte-like using a medium having an added component of 30 ng/mL human fibroblast growth factor 4 (FGF4), 50 ng/mL hepatocyte growth factor (HGF) and 50 ng/mL epidermal growth factor (EGF) during the eleventh to twentieth day, and changing the inducing medium every 2 to 3 days. The above culturing steps were all conducted by placing the apparatus for cell colony culture *in vitro* into an incubator with a condition of 37°C, 5% CO₂, and saturated humidity; 1.5 mL of medium was added into each well each time.

The above differentiated cell by inducing human embryonic stem cell H9 was able to express hepatic specific gene AFP, secreting albumin, by which could determine the human embryonic stem cell H9 had directed differentiated into hepatocyte-like.

### 2) directed inducing differentiation by co-culturing human embryonic stem cell H9 in situ

The human embryonic stem cell H9 cultured in step 1), after being observed forming 8×8 homogeneous colonies having a diameter of 1000 µm in the micro-patterned cavity, was subjecting to inducing differentiated culture by *in situ* co-culture in the apparatus for cell colony culture *in vitro.* The hepatocyte co-culture was taken as an example herein. The human embryonic stem cell H9 cultured in step 1) was subjected to inducing differentiated culture by *in situ* co-culture in the apparatus for cell colony culture *in vitro* prepared in Example 1, specific steps thereof were shown below:
Human hepatic stellate cells (cat#5300, Sciencell) were cultured with a DMEM medium added with 10% FBS and 200 U/mL penicillin/streptomycin in an incubator having a condition of 37°C, 5% CO₂, and saturated humidity.

Before starting co-culture with hepatic stellate cell the upper micro-patterned template was removed using a forceps, the culture plate was rinsed twice using a full medium for human embryonic stem cell H9, by this time the substrate hydrogel and micro-patterned colony of human embryonic stem cell H9 were both retained in the culture plate. The hepatic stellate cell was re-suspended in the full medium for human embryonic stem cell H9, and evenly seeded with a cell density of 2.5×10⁴ cell/cm² in the culture plate. After changing the medium the next day, the hepatic stellate cell preferably adhered to the substrate hydrogel, by which an environment for co-culturing the two kinds of cell were formed surrounding the colony of human embryonic stem cell H9.

After 6 days thereof, the human embryonic stem cell H9 was induced to a directed differentiation towards hepatocyte-like using an inducing medium, whose components were: 50% IMDM medium and 50% F12 NUT-MIX, added with 7 µg/mL insulin, 15 µg/mL transferrin, 450 µmol/L thioglycerol, 5 mg/mL bovine serum albumin (BSA), 50 ng/mL FGF4, 50 ng/mL HGF and 50 ng/mL EGF; and the inducing medium was changed every 2 to 3 days. The above culturing steps were all conducted by placing the apparatus for cell colony culture *in vitro* into an incubator with a condition of 37°C, 5% CO₂, and saturated humidity; 1.5 mL of medium was added into each well each time.

The above differentiated cell by inducing human embryonic stem cell H9 was able to express hepatic specific gene AFP, secreting albumin, by which could determine the human embryonic stem cell H9 had directed differentiated into hepatocyte-like.

### Example 4 formation of a multilayer micro-cluster of hESC using the apparatus for cell colony culture in vitro prepared in Example 1

hESC was cultured as stated in Example 3. After conventional digestion, cutting, centrifuging and collection, the human embryonic stem cells H9 (Wicell) having a confluence of 60 to 70 percentage conventionally-cultured in a six-well culture plate were further digested into single cells using a digestive enzyme such as Accutase (sigma). After cell counting, the single cells were seeded with a cell density of 2 × 10⁶ cell/mL into the apparatus for cell colony culture *in vitro.* At the same time 10 µg/mL ROCK blocker Y27632 was added into the medium to improve survival rate of hESC single cell. A homogeneous multilayer micro-cluster of hESC with high totipotency was then formed after 24 hours, the result thereof was shown in Fig. 8. As can be seen from Fig. 8, those cells with positive staining of alkaline phosphatase indicated an excellent totipotency of stem cell.

### Industrial applicability

The apparatus for cell colony culture *in vitro* of the present disclosure has controllable colony shape and size, adjustable physical and chemical property of the culture substrate, which may effectively achieve various culture methods such as a monolayer cell colony, a multilayer cell cluster, and cell co-culture, and may be used in regulating self-renewal and directed differentiation of totipotent stem cell and other kinds of stem cell.

Reference throughout this specification to "an embodiment," "some embodiments," "one embodiment", "another example," "an example," "a specific example," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example," "in an example," "in a specific example," or "in some examples," in various places throughout this specification are not necessarily referring to the same embodiment or example of the present disclosure. Furthermore, the particular features, structures, materials, or characteristics may be combined in any suitable manner in one or more embodiments or examples.

Although explanatory embodiments have been shown and described, it would be appreciated by those skilled in the art that the above embodiments can not be construed to limit the present disclosure, and changes, alternatives, and modifications can be made in the embodiments without departing from spirit, principles and scope of the present disclosure.

## Claims

1. An apparatus for cell colony culture *in vitro,* comprising:
a micro-patterned plate having a micro-patterned cavity;
an adhesive hydrogel, formed on a lower surface of the micro-patterned plate,
wherein
the micro-patterned cavity defines a growth space for cell colony.

2. The apparatus for cell colony culture *in vitro* of claim 1, further comprising:
a hard substrate, located on a lower surface of the adhesive hydrogel.

3. The apparatus for cell colony culture *in vitro* of claim 1, further comprising:
a multi-well culture plate, located under the adhesive hydrogel and embedded in the micro-patterned plate.

4. The apparatus for cell colony culture *in vitro* of claim 1, wherein the adhesive hydrogel is formed by cross-linking at least one selected from a group consisting of a natural biomaterial and a synthetic biomaterial.

5. The apparatus for cell colony culture *in vitro* of claim 4, wherein the natural biomaterial is at least one selected from a group consisting of gelatin, gelatin derivative, alginate, alginate derivative, agar, matrigel, collagen, proteoglycan, glycoprotein, hyaluronic acid, laminin and fibronectin.

6. The apparatus for cell colony culture *in vitro* of claim 4, wherein the synthetic biomaterial is at least one selected from a group consisting of polypropylene, polystyrene, polyacrylamide, polylactic, poly-hydroxyl acid, polylactic-alkyd copolymer, poly-dimethylsiloxane, polyanhydride poly-acid-ester, polyamide, polyamino acid, polyacetal, polycyanoacrylate, polyurethane, polypyrrole, polyester, polymethacrylate, polyethylene, polycarbonate and polyepoxyethane.

7. The apparatus for cell colony culture *in vitro* of claim 1, wherein the micro-patterned plate is formed by poly(methyl methacrylate).

8. The apparatus for cell colony culture *in vitro* of claim 1, wherein the adhesive hydrogel is formed from gelatin-methyl methacrylate and a photoinitiator, and the photoinitiator is 2-hydroxy-4-(2-hydroxyethoxy)-2-methyl propiophenon.

9. The apparatus for cell colony culture *in vitro* of claim 8, wherein the gelatin-methyl methacrylate is a polymer of gelatin and methyl methacrylate at a ratio of 1 g of gelatin against 1 mL of methyl methacrylate.

10. The apparatus for cell colony culture *in vitro* of claim 8, wherein the adhesive hydrogel is prepared by the following steps:
dissolving gelatin-methyl methacrylate in DMEM medium to obtain a gelatin-methyl methacrylate solution, wherein a content of gelatin-methyl methacrylate in the gelatin-methyl methacrylate solution is 5 g of gelatin- methyl methacrylate per 100 mL of the gelatin-methyl methacrylate solution;
dissolving the photoinitiator in absolute ethanol to obtain a photoinitiator solution, wherein a content of the photoinitiator in the photoinitiator solution is 10 g of the photoinitiator per 100 mL of the photoinitiator solution;
mixing the photoinitiator solution and the gelatin-methyl methacrylate solution at a volume ratio of 1:20; and
cross-linking gelatin-methyl methacrylate using ultraviolet ray to obtain the adhesive hydrogel.

11. The apparatus for cell colony culture *in vitro* of claim 1, the adhesive hydrogel comprising at least one selected from a group consisting of extracellular matrix component, growth factor and chemical signaling molecule.

12. The apparatus for cell colony culture *in vitro* of claim 1, wherein the extracellular matrix is coated on an upper surface of the adhesive hydrogel.

13. The apparatus for cell colony culture *in vitro* of claim 11, wherein the extracellular matrix component is at least one selected from a group consisting of collagen, gelatin, matrigel, proteoglycan, glycoprotein, hyaluronic acid, laminin and fibronectin.

14. The apparatus for cell colony culture *in vitro* of claim 11, wherein the adhesive hydrogel is fitted with the growth factor and the chemical signal molecule.

15. The apparatus for cell colony culture *in vitro* of claim 1, wherein the adhesive hydrogel has a thickness of 1-100 µm.

16. A usage of the apparatus for cell colony culture *in vitro* of any one of claims 1-15 in regulating the self-renewal of stem cell.

17. A usage of the apparatus for cell colony culture *in vitro* of any one of claims 1-15 in regulating the directed differentiation of stem cell.

18. An apparatus for cell colony culture *in vitro,* comprising:
a micro-patterned plate, having a plurality of micro-patterned cavities; and
an adhesive hydrogel, located on a lower surface of the micro-patterned plate;
wherein
the adhesive hydrogel and the micro-patterned plate are cross-linked and adhered integrally, and the shape and volume of each micro-patterned cavity of the micro-patterned plate determine a physical growth space of cell colony.

19. The apparatus for cell colony culture *in vitro* of claim 18, further comprising a hard substrate, located under the adhesive hydrogel.

20. The apparatus for cell colony culture *in vitro* of claim 18, further comprising:
a multi-well culture plate, located under the adhesive hydrogel and embedded in the micro-patterned plate.

21. The apparatus for cell colony culture *in vitro* of any one of claims 18-20, wherein the adhesive hydrogel is prepared using a natural biomaterial and/or synthetic biomaterial, which is cross-linkable to form a gel,
the natural biomaterial is at least one selected from a group consisting of gelatin, gelatin derivative, alginate, alginate derivative, agar, matrigel, collagen, proteoglycan, glycoprotein, hyaluronic acid, laminin and fibronectin;
the synthetic biomaterial is at least one selected from a group consisting of polypropylene, polystyrene, polyacrylamide, polylactic, poly-hydroxyl acid, polylactic-alkyd copolymer, poly-dimethylsiloxane, polyanhydride, poly-acid-ester, polyamide, polyamino acid, polyacetal, polycyanoacrylate, polyurethane, polypyrrole, polyester, polymethacrylate, polyethylene, polycarbonate and polyepoxyethane.

22. The apparatus for cell colony culture *in vitro* of any one of claims 18-21, wherein a material used for the micro-patterned plate is poly(methyl methacrylate); the material used for the adhesive hydrogel consists of gelatin-methyl methacrylate and a photoinitiator 2-hydroxy-4-(2-hydroxyethoxy)-2 methyl propiophenon;
the gelatin-methyl methacrylate is a polymer of gelatin and methyl methacrylate,
a ratio between gelatin and methyl methacrylate is 1 g of gelatin against 1 mL of methyl methacrylate.

23. The apparatus for cell colony culture *in vitro* of claim 22, wherein the adhesive hydrogel is prepared by the following steps:
(1) dissolving gelatin-methyl methacrylate in DMEM medium, to obtain a gelatin-methyl methacrylate solution , wherein a content of gelatin-methyl methacrylate in the gelatin-methyl methacrylate solution is 5 g of gelatin- methyl methacrylate per 100 mL of the gelatin-methyl methacrylate solution;
(2) dissolving the photoinitiator 2-hydroxy-4-(2-hydroxyethoxy)-2 methyl propiophenon in absolute ethanol, to obtain a photoinitiator solution, wherein a content of 2-hydroxy-4-(2-hydroxyethoxy)-2 methyl propiophenon in the photoinitiator solution is 10 g of 2-hydroxy-4-(2-hydroxyethoxy)-2 methyl propiophenon per 100 mL of the photoinitiator solution;
3) mixing the photoinitiator solution and the gelatin-methyl methacrylate solution at a volume ratio of 1:20.

24. The apparatus for cell colony culture *in vitro* of any one of claims 18-23, wherein extracellular matrix component is coated on an upper surface of the adhesive hydrogel; the extracellular matrix component comprises collagen, gelatin, matrigel, proteoglycan, glycoprotein, hyaluronic acid, laminin and fibronectin; and/or,
the adhesive hydrogel is fitted with growth factor and chemical signal molecule.

25. The apparatus for cell colony culture *in vitro* of any one of claims 18-24, wherein the adhesive hydrogel has a thickness of 1-100 µm.

26. A method for regulating the self-renewal of stem cell, comprising:
culturing the stem cell using the apparatus for cell colony culture *in vitro* of any one of claims 18-25.

27. A method for regulating the directed differentiation of stem cell, comprising:
culturing the stem cell using the apparatus for cell colony culture *in vitro* of any one of claims 18-25.

28. A method for forming a monolayer micro-pattern of cell, comprising:
culturing the cell using the apparatus for cell colony culture *in vitro* of any one of claims 18-25, to obtain the monolayer micro-pattern of cell,
wherein the cell is at least one selected from stem cell and adult cell.

29. A method for forming a multilayer micro-cluster of cell, comprising:
culturing the cell using the apparatus for cell colony culture *in vitro* of any one of claims 18-25, to obtain the multilayer micro-cluster of cell,
wherein the cell is at least one selected from stem cell and adult cell.

## Amended claims

### Amended claims under Art. 19.1 PCT

**1.** An apparatus for cell colony culture *in vitro,* comprising:
a micro-patterned plate having a micro-patterned cavity;
a viscous hydrogel, formed on a lower surface of the micro-patterned plate,
wherein
the micro-patterned cavity defines a growth space for cell colony,
the micro-patterned plate is formed by poly(methyl methacrylate).

**2.** The apparatus for cell colony culture *in vitro* of claim 1, further comprising:
a hard substrate, located on a lower surface of the viscous hydrogel.

**3.** The apparatus for cell colony culture *in vitro* of claim 1, further comprising:
a multi-well culture plate, located under the viscous hydrogel and embedded in the micro-patterned plate.

**4.** The apparatus for cell colony culture *in vitro* of claim 1, wherein the viscous hydrogel is formed by cross-linking at least one selected from a group consisting of a natural biomaterial and a synthetic biomaterial.

**5.** The apparatus for cell colony culture *in vitro* of claim 4, wherein the natural biomaterial is at least one selected from a group consisting of gelatin, gelatin derivative, alginate, alginate derivative, agar, matrigel, collagen, proteoglycan, glycoprotein, hyaluronic acid, laminin and fibronectin.

**6.** The apparatus for cell colony culture *in vitro* of claim 4, wherein the synthetic biomaterial is at least one selected from a group consisting of polypropylene, polystyrene, polyacrylamide, polylactic, poly-hydroxyl acid, polylactic-alkyd copolymer, poly-dimethylsiloxane, polyanhydride poly-acid-ester, polyamide, polyamino acid, polyacetal, polycyanoacrylate, polyurethane, polypyrrole, polyester, polymethacrylate, polyethylene, polycarbonate and polyepoxyethane.

**8.** The apparatus for cell colony culture *in vitro* of claim 1, wherein the viscous hydrogel is formed from gelatin-methyl methacrylate and a photoinitiator, and the photoinitiator is 2-hydroxy-4-(2-hydroxyethoxy)-2-methyl propiophenon.

**9.** The apparatus for cell colony culture *in vitro* of claim 8, wherein the gelatin-methyl methacrylate is a polymer of gelatin and methyl methacrylate at a ratio of 1 g of gelatin against 1 mL of methyl methacrylate.

**10.** The apparatus for cell colony culture *in vitro* of claim 8, wherein the viscous hydrogel is prepared by the following steps:
dissolving gelatin-methyl methacrylate in DMEM medium to obtain a gelatin-methyl methacrylate solution, wherein a content of gelatin-methyl methacrylate in the gelatin-methyl methacrylate solution is 5 g of gelatin- methyl methacrylate per 100 mL of the gelatin-methyl methacrylate solution;
dissolving the photoinitiator in absolute ethanol to obtain a photoinitiator solution, wherein a content of the photoinitiator in the photoinitiator solution is 10 g of the photoinitiator per 100 mL of the photoinitiator solution;
mixing the photoinitiator solution and the gelatin-methyl methacrylate solution at a volume ratio of 1:20; and
cross-linking gelatin-methyl methacrylate using ultraviolet ray to obtain the viscous hydrogel.

**11.** The apparatus for cell colony culture *in vitro* of claim 1, the viscous hydrogel comprising at least one selected from a group consisting of extracellular matrix component, growth factor and chemical signaling molecule.

**12.** The apparatus for cell colony culture *in vitro* of claim 1, wherein the extracellular matrix is coated on an upper surface of the viscous hydrogel.

**13.** The apparatus for cell colony culture *in vitro* of claim 11, wherein the extracellular matrix component is at least one selected from a group consisting of collagen, gelatin, matrigel, proteoglycan, glycoprotein, hyaluronic acid, laminin and fibronectin.

**14.** The apparatus for cell colony culture *in vitro* of claim 11, wherein the viscous hydrogel is fitted with the growth factor and the chemical signal molecule.

**15.** The apparatus for cell colony culture *in vitro* of claim 1, wherein the viscous hydrogel has a thickness of 1-100 µm.

**16.** A usage of the apparatus for cell colony culture *in vitro* of any one of claims 1-15 in regulating the self-renewal of stem cell.

**17.** A usage of the apparatus for cell colony culture *in vitro* of any one of claims 1-15 in regulating the directional differentiation of stem cell.

**18.** An apparatus for cell colony culture *in vitro,* comprising:
a micro-patterned plate, having a plurality of micro-patterned cavities; and
a viscous hydrogel, located on a lower surface of the micro-patterned plate;
wherein
the viscous hydrogel and the micro-patterned plate are cross-linked and adhered integrally, and the shape and volume of each micro-patterned cavity of the micro-patterned plate determine a physical growth space of cell colony, and
a material used for the micro-patterned plate is poly(methyl methacrylate).

**19.** The apparatus for cell colony culture *in vitro* of claim 18, further comprising a hard substrate, located under the viscous hydrogel.

**20.** The apparatus for cell colony culture *in vitro* of claim 18, further comprising:
a multi-well culture plate, located under the viscous hydrogel and embedded in the micro-patterned plate.

**21.** The apparatus for cell colony culture *in vitro* of any one of claims 18-20, wherein the viscous hydrogel is prepared using a natural biomaterial and/or synthetic biomaterial, which is cross-linkable to form a gel,
the natural biomaterial is at least one selected from a group consisting of gelatin, gelatin derivative, alginate, alginate derivative, agar, matrigel, collagen, proteoglycan, glycoprotein, hyaluronic acid, laminin and fibronectin;
the synthetic biomaterial is at least one selected from a group consisting of polypropylene, polystyrene, polyacrylamide, polylactic, poly-hydroxyl acid, polylactic-alkyd copolymer, poly-dimethylsiloxane, polyanhydride, poly-acid-ester, polyamide, polyamino acid, polyacetal, polycyanoacrylate, polyurethane, polypyrrole, polyester, polymethacrylate, polyethylene, polycarbonate and polyepoxyethane.

**22.** The apparatus for cell colony culture *in vitro* of any one of claims 18-21, wherein the material used for the viscous hydrogel consists of gelatin-methyl methacrylate and a photoinitiator 2-hydroxy-4-(2-hydroxyethoxy)-2 methyl propiophenon;
the gelatin-methyl methacrylate is a polymer of gelatin and methyl methacrylate,
a ratio between gelatin and methyl methacrylate is 1 g of gelatin against 1 mL of methyl methacrylate.

**23.** The apparatus for cell colony culture *in vitro* of claim 22, wherein the viscous hydrogel is prepared by the following steps:
(1) dissolving gelatin-methyl methacrylate in DMEM medium, to obtain a gelatin-methyl methacrylate solution , wherein a content of gelatin-methyl methacrylate in the gelatin-methyl methacrylate solution is 5 g of gelatin- methyl methacrylate per 100 mL of the gelatin-methyl methacrylate solution;
(2) dissolving the photoinitiator 2-hydroxy-4-(2-hydroxyethoxy)-2 methyl propiophenon in absolute ethanol, to obtain a photoinitiator solution, wherein a content of 2-hydroxy-4-(2-hydroxyethoxy)-2 methyl propiophenon in the photoinitiator solution is 10 g of 2-hydroxy-4-(2-hydroxyethoxy)-2 methyl propiophenon per 100 mL of the photoinitiator solution;
3) mixing the photoinitiator solution and the gelatin-methyl methacrylate solution at a volume ratio of 1:20.

**24.** The apparatus for cell colony culture *in vitro* of any one of claims 18-23, wherein extracellular matrix component is coated on an upper surface of the viscous hydrogel; the extracellular matrix component comprises collagen, gelatin, matrigel, proteoglycan, glycoprotein, hyaluronic acid, laminin and fibronectin; and/or,
the viscous hydrogel is fitted with growth factor and chemical signal molecule.

**25.** The apparatus for cell colony culture *in vitro* of any one of claims 18-24, wherein the viscous hydrogel has a thickness of 1-100 µm.

**26.** A method for regulating the self-renewal of stem cell, comprising:
culturing the stem cell using the apparatus for cell colony culture *in vitro* of any one of claims 18-25.

**27.** A method for regulating the directional differentiation of stem cell, comprising:
culturing the stem cell using the apparatus for cell colony culture *in vitro* of any one of claims 18-25.

**28.** A method for forming a monolayer micro-pattern of cell, comprising:
culturing the cell using the apparatus for cell colony culture *in vitro* of any one of claims 18-25, to obtain the monolayer micro-pattern of cell,
wherein the cell is at least one selected from stem cell and adult cell.

**29.** A method for forming a multilayer micro-cluster of cell, comprising:
culturing the cell using the apparatus for cell colony culture *in vitro* of any one of claims 18-25, to obtain the multilayer micro-cluster of cell,
wherein the cell is at least one selected from stem cell and adult cell.
